# EUROPÄISCHE PATENTSCHRIFT

(11) **EP 0 338 437 B1**
(45) Veröffentlichungstag und Bekanntmachung des Hinweises auf die Patenterteilung: **15.02.1995**
(21) Anmeldenummer: 89106628.4
(22) Anmeldetag: 13.04.1989
(51) Int. Cl.: C07K 14/09, A61K 38/02

(54) **Synthetische Vakzine gegen die Maul- und Klauenseuche und Verfahren zu deren Herstellung**
Synthetic vaccins against foot and mouth disease and their preparation process
Vaccins synthétiques contre la fièvre aphteuse et leur procédé de préparation

(30) Priorität: 22.04.1988 DE 3813821
(43) Veröffentlichungstag der Anmeldung: 25.10.1989
(73) Patentinhaber: HOECHST AKTIENGESELLSCHAFT, 65926 Frankfurt am Main (DE)
(72) Erfinder: Wiesmüller, Karl-Heinz, D-7400 Tübingen (DE); Hess, Günter, Dr. Dr., D-4500 Koblenz (DE); Jung, Günther, Prof. Dr., D-7400 Tübingen (DE)

(56) Entgegenhaltungen:
- EP-A- 0 210 412
- PEPTIDE - PROCEEDINGS OF THE 10th AMERICAN PEPTIDE SYMPOSIUM, St Louis, 23.-28.Mai 1987, Seiten 553-554, ESCOM, Leiden, NL; A.YU. SUROVOY et al.:" Mimickingprotective epitopes of foot and mouth disease virus with synthetic peptides " *Insgesamt *
- EXPERIENTIA, Band 42, 1986, Seiten 521-531, Birkhäuser Verlag, Basel, CH; G.H.
- WERNER et al.: "Immunomodulating peptides " * Seite 522, (Lipopeptides )- Seite524, Spalte 1 *
- Symposium on synthetic peptides as antigens, London , 4.-6. Juni 1985, Seiten184-199, Wiley & Sons, Chichester, GB; M. SELA et al. :" SYnthetic peptideswith antigenic specificity for bacterial toxins " * Seite 184 (Zusammenfassung); Seiten 186-187 ( Synthetische Vakzine); Seite 192, Spalte 1 und Figur4 ;Seite 197 , Spalte 2- Seite 198, Spalte 1 *
- PROC. NATL. ACAD. SCI. USA, Band 82, Januar 1985, Seiten 178-182, Washington,US; H.M. GEYSEN et al. :"Small peptides induce antibodies with a sequence andstructural requirement for binding antigen comparable to antibodies raisedagainst the native protein " * Seite 179 , Figur 1 *
- VACCINE, Band 7, Februar 1989, Seiten 29-33, Butterworth & Co. Ltd, Guildford,GB; K.-H. WIESMULLER et al. :"Novel low-molecular-weigth synthetic vaccineagainst foot-and-mouth disease containing a potent b-cell and macrophageactivator " * Insgesamt *

## Beschreibung

Die vorliegende Erfindung betrifft Vakzine gegen die Maul- und Klauenseuche sowie Verfahren zu deren Herstellung.

Die Maul- und Klauenseuche (MKS) verursacht trotz bereits lange verfügbarer Impfstoffe in der Viehzucht große Verluste. Ein Grund für das Auftreten von Maul- und Klauenseuche in der heutigen Zeit ist die Unsicherheit von klassischen Impfstoffen, die abgetötete bzw. inaktivierte MKS-Viren enthalten: Die Inaktivierung der Viren ist mitunter nicht vollständig, so daß zu "post-vaccinalen" Ausbrüchen der MKS kommen kann (vgl. Böhm, Strohmaier, Tierärztl. Umschau 39, 3 - 8 (1984)). Diese Gefahr ist bei synthetischen MKS-Vakzinen nicht vorhanden, weil bei letzteren nur Partialsequenzen von bestimmten Virusproteinen verwendet werden, die nicht die Funktion eines intakten Virus haben.

Es existieren zwar bereits synthetische MKS-Vakzine (vgl. Europäische Patentanmeldung 0 204 480), die aber noch verbesserungsbedürftig sind.

Es wurde nun gefunden, daß unter Verwendung von Membranankerverbindungen und bestimmten Partialsequenzen des MKS-Virus besonders wirksame MKS-Vakzine hergestellt werden können. In der deutschen Offenlegungsschrift DE 35 46 150 A1 wird zwar als eine von vielen Anwendungsmöglichkeiten von Membrananker-Wirkstoffkonjugaten die Herstellung von synthetischen Vakzinen erwähnt, es war aber nicht zu erwarten, daß die Konjugate aus Membranankerverbindungen und Partialsequenzen des MKS-Virus (Membranankerwirkstoffkonjugate) die gefundene außergewöhnliche Wirksamkeit bei Applikation relativ geringer Mengen an Vakzine aufweisen.

Weiterhin zeichnen sich die genannten Vakzine überraschenderweise dadurch aus, daß sie bereits nach einmaliger Applikation einen ausreichenden Impfschutz bieten. Gegenüber herkömmlichen Vakzinen haben sie zudem den Vorteil, ohne Kühlung praktisch unbegrenzt haltbar zu sein.

Erfindungsgegenstand ist demzufolge eine synthetische Vakzine, dadurch gekennzeichnet, daß die Vakzine eine Wirkung gegen die Maul- und Klauenseuche aufweist und aus einem Konjugat aus einer Membranankerverbindung und einer Partialsequenz des Maul- und Klauenseuche-Virus, die kovalent miteinander verknüpft sind, besteht, wobei die Membranankerverbindung eine Struktur der nachfolgenden Formeln besitzt 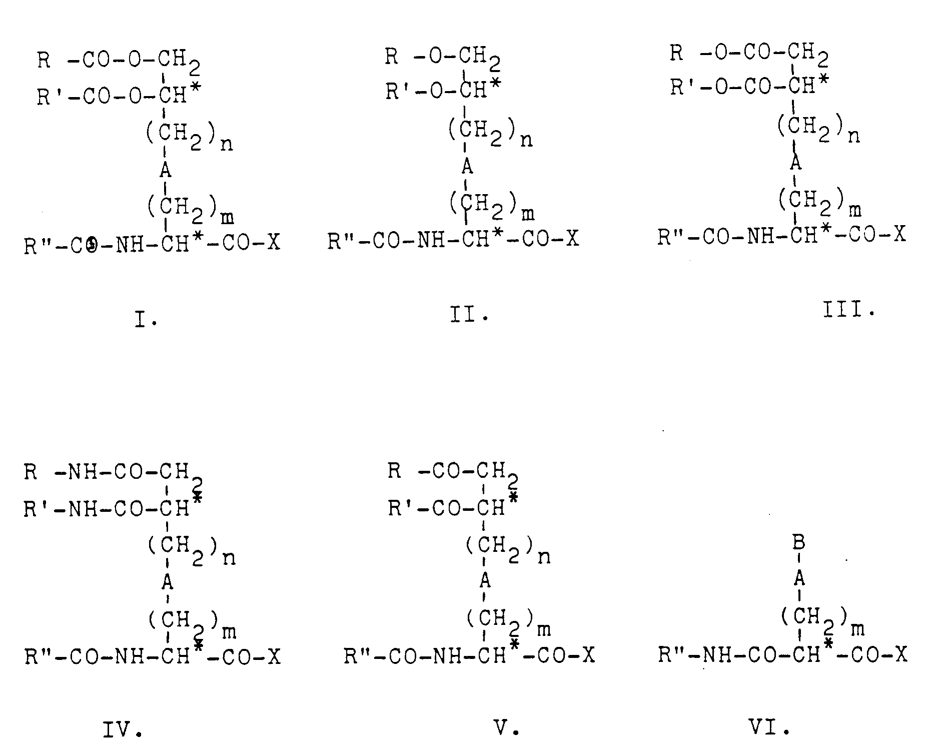 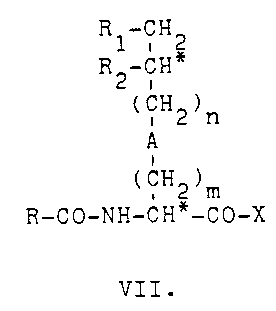
in denen A Schwefel, Sauerstoff, Disulfid (-S-S-),
Methylen (-CH₂-) oder -NH- sein kann;
n = 0 bis 5, m = 1 oder 2;
C* ein asymmetrisches Kohlenstoffatom mit R- oder S-Konfiguration,
R, R' und R'' gleich oder verschieden sind und Wasserstoff oder eine Alkyl-, Alkenyl- oder Alkinylgruppe mit 7 bis 25 Kohlenstoffatomen ist, welche mit Hydroxy-, Amino-, Oxo-, Acyl-, Alkyl- oder Cycloalkylgruppen substituiert sein kann, B in Formel VI die Bedeutung jedes der in den Formeln I - V aufgeführten -(CH₂)ₙ-(substituiertes Alkyl)-Reste haben kann und R₁ und R₂ gleich oder verschieden sind und dieselben Bedeutungen wie R, R' und R'' haben, aber auch -OR, -O-COR, -COOR, NHCOR oder -CONHR sein können, wobei X eine Kette von 1 bis 10 Aminosäuren ist, an die die Partialsequenz des Virus gebunden ist.

Membranankerverbindungen sind Verbindungen, die in biologische oder künstliche Membranen einschleusbar sind.

Weitere Erläuterungen zu den Membranankerverbindungen finden sich in der bereits zitierten deutschen Offenlegungsschrift 35 46 150 und in G. Jung et al. in "Peptides, Structure and Function", V.J. Hruby and D.H. Rich, Seiten 179 bis 182, Pierce Chem. Co. Rockford, Illinois, (1983).

Von den Membranankerverbindungen sind als Beispiele besonders hervorzuheben:
In bakteriellem Lipoprotein vorkommende N-Termini, wie z.B.: Y-Ser-Ser-Ser-Asn, Y-Ile-Leu-Leu-Ala, Y-Ala-Asn-Asn-Gln, Y-Asn-Ser-Asn-Ser, Y-Gly-Ala-Met-Ser, Y-Gln-Ala-Asn-Tyr, Y-Gln-Val-Asn-Asn, Y-Asp-Asn-Ser-Ser, wobei Y eine der unter Formel I bis VII aufgeführten Reste sein kann. Diese Lipopentapeptide können auch in verkürzter Form (Lipodi, Lipotri oder Lipotetrapeptide) als Membranankerverbindung eingesetzt werden. Ganz besonders bevorzugt ist N-Palmitoyl-S-[2,3(bispalmitoyloxy)propyl]-cysteinyl-seryl-serin (Pam₃Cys-Ser-Ser), N-Palmitoyl-S-[2,3-(bispalmitoyloxy)propyl]-cysteinyl-seryl-glycin und N-Palmitoyl-S-[2,3-(bispalmitoyloxy)propyl]-cysteinyl-alanyl-D-isoglutamin. Beispiele weiterer bevorzugter Membranankerverbindungen finden sich in der DE-OS 35 46 150.

Als Partialsequenzen des MKS-Virus, die an die Membranankerverbindung gebunden werden, können viele verschiedene Partialsequenzen eingesetzt werden. Bevorzugt sind die Partialsequenzen:
Partialsequenz -(134-154)
Partialsequenz -(135-154)
Partialsequenz -(134-158)
Partialsequenz -(134-160)
Partialsequenz -(141-160)
Partialsequenz -(141-158)
Partialsequenz -(200-213)
Partialsequenz -(200-210)
Partialsequenz -(161-180)
wobei die Sequenzen aller bekannten Serotypen und Subtypen verwendet werden können. Als beispielhafte Serotypen seien in diesem Zusammenhang angegeben: 
Serotyp A:      134                          160
A5 Westerwald    NKYSTGGP--RRGDMGSAAARAAKQLP
                 161                    180
 
ASFNYGAIRAITIHELLVRM
200 213
RHKQKIIAPARQLL
Besonders geeignet sind synthetische Vakzine, die aus einer Mischung von Peptiden aus verschiedenen Sero- und/oder Subtypen des Maul- und Klauenseuche-Virus bestehen, die jeweils kovalent an die Membranankerverbindung(en) gebunden sind.

Besonders bevorzugt sind synthetische Vakzine, die aus einer Mischung von Sequenzen VP1 134-160 der Serotypen O, A, C, gebunden an die Membranankerverbindung N-Palmitoyl-S-[2,3-(bispalmitoyloxy)propyl]-cysteinyl-seryl-serin, bestehen.

Bei Verwendung der Sequenz 134-154 vom Serotyp O und der Sequenz 134-155 vom Serotyp A kann dieselbe, soweit sie C-terminales Lysin enthält, über die ε-Aminogruppe mit der Membranankerverbindung kovalent verknüpft werden.

Als besonders geeignet haben sich erfindungsgemäße synthetische Vakzine erwiesen, die die Partialsquenz des MKS-Virus VP 1(135-154) enthalten.

Besonders bevorzugt ist weiterhin eine Vakzine bestehend aus N-Palmitoyl-S-[2,3-(bispalmitoyloxy)-propyl]-cysteinyl-seryl -seryl-VP 1 (135-154), d.h. die Verbindung der nachstehenden Formel.

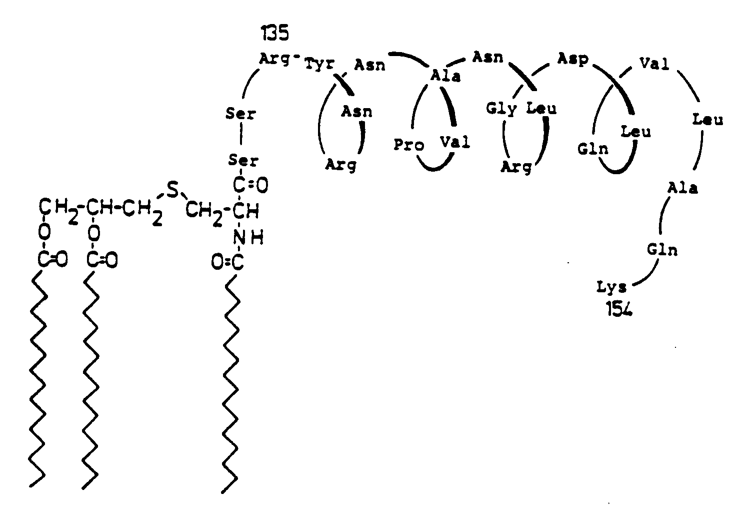

Die Membranankerverbindungen können grundsätzlich als R,S-, R,R-Diastereomere oder als Diastereomerengemisch vorliegen. Es hat sich allerdings gezeigt, daß die Vakzinen, die eine R,R-diastereomere Membranankerverbindung enthalten, eine besonders hohe Wirksamkeit zeigen.

Erfindungsgegenstand ist weiterhin ein Verfahren zur Herstellung einer synthetischen Vakzine, das dadurch gekennzeichnet ist, daß Partialsequenzen des MKS-Virus durch eine Konjugationsreaktion an die Membranankerverbindung gebunden wird. Die Konjugationsreaktion kann z.B. eine Kondensation, Addition, Substitution, Oxidation oder Disulfidbildung sein. Bevorzugte Konjugationsmethoden sind in Beispiel 1 wiedergegeben. Weitere Konjugationsmethoden sind in der bereits zitierten deutschen Offenlegungsschrift 35 46 150 beschrieben.

Die Herstellung der Membranankerverbindungen ist ebenfalls in der zuletzt genannten deutschen Offenlegungsschrift ausführlich beschrieben.

Die gegebenenfalls nötige Trennung der Diastereomeren kann nach unterschiedlichen Methoden, wie z.B. in Hoppe-Seyler's Z. Physiolog. Chem. 364 (1983) 593 beschrieben erfolgen. In Beispiel 2 ist ein bevorzugtes Trennverfahren beschrieben.

Der Aufbau der Partialsequenzen der jeweiligen MKS-Proteine kann auf unterschiedliche, literaturbekannte Weise erfolgen, vgl. z.B. Wünsch et al. in Houben-Weyl, Bd. 15/1,2, Stuttgart, Thieme-Verlag oder Wünsch in Angew. Chem. 83 (1971), 773, E. Gross und J. Meienhofer (Herausgeb.), The Peptides, Vol. 1 (1979), 2 (1979), 3 (1981) und 5 (1983) Academic Press, New York oder die deutsche Offenlegungsschrift 35 46 150. In Beispiel 3 wird ein bevorzugtes Verfahren zur Herstellung einer Partialsequenz und eines Konjugats näher erläutert.

Weiterhin gehören zum Erfindungsgegenstand pharmazeutische oder veterinärmedizinische Zubereitungen, die einen Gehalt an Konjugat aus Membranankerverbindung und Partialsequenz eines MKS-Virus aufweisen. Normalerweise werden zusätzlich neben einem Lösungsmittel keine zusätzlichen Hilfs- und Trägerstoffe oder Adjuvanzien für die erfindungsgemäßen Zubereitungen benötigt. In manchen Fällen kann es aber sinnvoll sein, derartige Hilfs- und/oder Trägerstoffe sowie gegebenenfalls Adjuvanzien den erfindungsgemäßen Zubereitungen zuzusetzen. Das Mischen und Abfüllen der betreffenden Stoffe erfolgt nach dem Fachmann bekannten Verfahren.

Die Menge an Vakzine, die für eine sichere Immunisierung eines Tieres notwendig ist, hängt ab von der Tierart,der bzw. den Membranankerverbindungen und der bzw. den Partialsequenzen des MKS-Virus und ist im Einzelfall empirisch zu ermitteln. Für die sichere Immunisierung eines Meerschweinchens gegen den MKS-Virus-Serotyp O₁K genügt z.B. eine einmalige Verabreichung von ca. 100 - 500 »g von erfindungsgemäßer Vakzine, ohne weitere Hilfs- oder Trägerstoffe.

Weiterhin gehört zum Erfindungsgegenstand die Verwendung der beschriebenen Vakzine zur Erzeugung von Antikörpern in Säugetieren.

### Beispiel 1

### Konjugation von Peptiden/Proteinen mit Pam₃Cys-Ser-Ser-OSu bzw. Pam₃Cys-Ser-Ser-OH

1. Peptide und Proteine löslich in DMF
   2 »Mol Peptid/Protein werden in 0,5 - 1 ml DMF gelöst und 8 »Mol (9,2 mg) festes Pam₃Cys-Ser-Ser-OSu zugegeben. Durch leichtes Erwärmen und beschallen wird eine homogene Lösung erhalten und es werden 4 »Mol organische Base (N-Ethylmorpholin) zugegeben. Nach 12 h Rühren werden 1 - 2 ml Chloroform:Methanol (1:1) zugegeben und es wird 2 h im Eisbad gekühlt.
   Das Sediment wird mit 1 ml kaltem Chloroform:Methanol (1:1) gewaschen in tert.Butanol/Wasser (3:1) aufgenommen (evtl. beschallen) und lyophilisiert.
2. Peptide und Proteine löslich in Wasser
   2 »Mol Peptid/Protein werden in 0,8 ml Wasser gelöst und mit 4 »Mol (4,5 mg) Pam₃Cys-Ser-Ser-OH versetzt. Es wird gründlich beschallt bis eine Emulsion entsteht und ein pH-Wert von 5,0 bis 5,5 eingestellt. Nach Zugabe von 5 mg EDC (1-(3-Dimethylaminopropyl)-3-ethyl-carbodiimid-hydrochlorid), gelöst in 100 »l H₂O, wird 18 h bei Raumtemperatur gerührt und anschließend zweimal gegen je 1 l dest. H₂O dialysiert. Der Inhalt des Dialyseschlauches wird lyophilisiert.

### Beispiel 2

### Trennung der Diastereomeren von N-Palmitoyl-S-[2,3-(bis-palmitoyloxy)propyl]-cystein-tert.-butylester (Pam₃Cys-OBu^{t)}:

2 g Pam₃Cys-OBu^{t} werden in 10 ml Laufmittel, Dichlormethan/Essigester (20:1), gelöst und auf eine Säule (Länge 120 cm, Durchmesser 4 cm) gefüllt mit MN-Kieselgel 60, 0,063-0,2 mm/70 - 230 mesh ASTM, aufgetragen. Bei einer Tropfgeschwindigkeit von 2 Tr./sec werden 350 Fraktionen a 10 ml gesammelt und ein Aliquot jeder Fraktion nach Chromatographie auf Kieselgel 60-Platten in Dichlormethan/Essigester (20:1) und Anfärbung mit Chlor/TDM Reagenz auf Pam₃Cys-OBu^{t} geprüft.

Fraktionen 280 -315 enthalten das R,R-Diastereomere, Fraktionen 316 - 335 eine Mischung aus R,R und R,S und Fraktionen 336 - 354 das R,S Diastereomere von Pam₃Cys-OBu^{t}. Nach Abdampfen des Lösungsmittels am Rotationsverdampfer, Aufnehmen des Rückstandes in warmem tert.-Butanol und Lyophilisieren erhält man 600 mg R,R-, 370 mg Mischung aus R,R- und R,S- und 540 mg R,S-Pam₃Cys-OBu^{t}.

### Beispiel 3

### Synthese von N-Palmitoyl-S-[2,3-(bispalmitoyloxy)-propyl]-cysteinyl-seryl-seryl-VP 1 (135-154)

Die VP 1 Peptidsequenz des MKS-Virus Serotyp O₁K wurde durch Festphasen-Peptidsynthese synthetisiert. Es wurden Fmoc Aminosäuren benutzt. Folgende Seitenkettenschutzgruppen kamen zur Anwendung: Lys(Boc), His(Fmoc), Arg(Mtr), Ser(tBu), Asp(OtBu), Tyr(tBu). Ausgehend von 1 g p-Benzoyloxybenzylalkohol-Harz, beladen mit Fmoc-Lys(Boc)-OH (0,47 mmol/g) wurden folgende Synthesezyklen durchlaufen:

N-Aktivierung mit 55 % Piperidin in N-Methylpyrrolidon (1 x 2 Min, 1 x 5 Min), Präaktivierung von Fmoc-A-A-OH (1,5 mmol) in N-Methylpyrrolidon (6 ml) mit Diisopropylcarbodiimid (1,5 mmol) und 1-Hydroxybenzotriazol (1,5 mmol) mit anschließender Kupplung für 1,5 Std. Nach Waschen mit N-Ethylmorpholin (5 % in N-Methylpyrrolidon) wurden Präaktivierung und Kupplung wiederholt. Die Blockierung von nicht umgesetzten Aminogruppen wurde mit Acetanhydrid (2,5 mmol) und Diisopropylamin (1,2 mmol) in N-Methylpyrrolidon durchgeführt. Nach jedem Schritt wurde das Peptid-Harz mehrfach mit N-Methylpyrrolidon, Dichlormethan und erneut mit N-Methylpyrrolidon gewaschen.

Nach der Synthese der harzgebundenen MKS-Virus-Sequenz wurde ein Teil des Peptids durch Trifluoressigsäurespaltung gewonnen und überprüft mittels HPLC, MS, Aminosäurenanalyse, Analyse auf chiraler Phase sowie Sequenzanalyse. Nachdem 2 Serinreste an das harzgebundene Peptid gebunden wurden, erfolgte die Kupplung des Tripalmitoyl-S-glycerylcysteins. Nach 4 Stunden wurde 1 Äquivalent N-Methylmorpholin hinzugefügt und nach einer weiteren Stunde wurde das Lipopeptid-Harz gewaschen. Das Lipopeptid wurde vom Harz mittels 2 ml Trifluoressigsäure (mit 100 »l Thioanisol) innerhalb von 4 1/2 Stunden getrennt. Das Filtrat wurde eingedampft, der Rückstand mit Essigsäure aufgenommen und in kalten Ether gegeben. Das ausgefallene Lipopeptid wurde 3 x mit Ether gewaschen. Weitere Reinigung wurde erzielt durch Umkristallisation aus Trifluoroethanol/Chloroform im Verhältnis 1:3 mit kaltem Aceton und einigen Tropfen Wasser. Das Lipopeptid wurde aus tert.-Butanol/Wasser im Verhältnis 3:1 lyophilisiert.

### Beispiel 4

### Wirksamkeitstest:

Zufällig ausgewählte Meerschweinchen mit einem Gewicht von 450 bis 500 g wurden intramuskulär oder subkutan geimpft. 0,5 mg der lyophiliserten Vakzine (N-Palmitoyl-S-[(2R,R)-2,3-(bispalmitoyloxy)propyl]-cysteinyl-seryl-seryl-VPI(135-154) wurden emulsifiziert in 500 »l einer 1:1-Mischung aus 0,05 M Phosphatpuffer und Intralipid^{(R)} (Kabi Vitrum, Schweden). Die Mischung wurde für 10 s beschallt. Vier Tiere wurden mit dem MKS-Virus infiziert, indem ihnen in die linke Hinterpfote mindestens 500 Meerschweinchen-Einheiten eines virulenten O₁K FMD-Virus 21 Tage nach der Impfung subkutan injiziert wurden. Als Kontrollen wurden Tiere herangezogen, denen an Stelle des Impfstoffs die Membranankerverbindung, bzw. Phosphatpuffer injiziert worden war. Bei allen geimpften Tieren wurde ein hoher Titer neutralisierender Antikörper log₁₀SN₅₀ von 0,36 gefunden. Die Kontroll-Tiere hatten keinen Antikörper-Titer (Blindwert 0,17). Der Titer der neutralisierenden Antikörper wurde bestimmt als Logarithmus der Serum-Verdünnung, die notwendig war, um 50 % der Viruszellen in einer einlagigen Schicht von BHK-(Baby Hamster Kidney)-Zellen zu neutralisieren. Bei den geimpften Tieren konnten mittels Anti-Peptid ELISA-Assays (A₄₉₂) Antikörper nachgewiesen werden, was bei den nicht geimpften Tieren nicht möglich war. Geimpfte Tiere zeigten keine Sekundärläsionen, während alle nicht geimpften Tiere das Vollbild der Maul- und Klauenseuche-Infektion zeigten.

## Patentansprüche (Patentansprüche für folgende(n) Vertragsstaat(en): AT, BE, CH, DE, FR, GB, IT, LI, LU, NL, SE)

1. Synthetische Vakzine, dadurch gekennzeichnet, daß die Vakzine eine Wirkung gegen die Maul- und Klauenseuche aufweist und aus einem Konjugat aus einer Membranankerverbindung und einer Partialsequenz des Maul- und Klaueseuche-Virus, die kovalent miteinander verknüpft sind, besteht, wobei die Membranankerverbindung eine Struktur der nachfolgenden Formeln besitzt 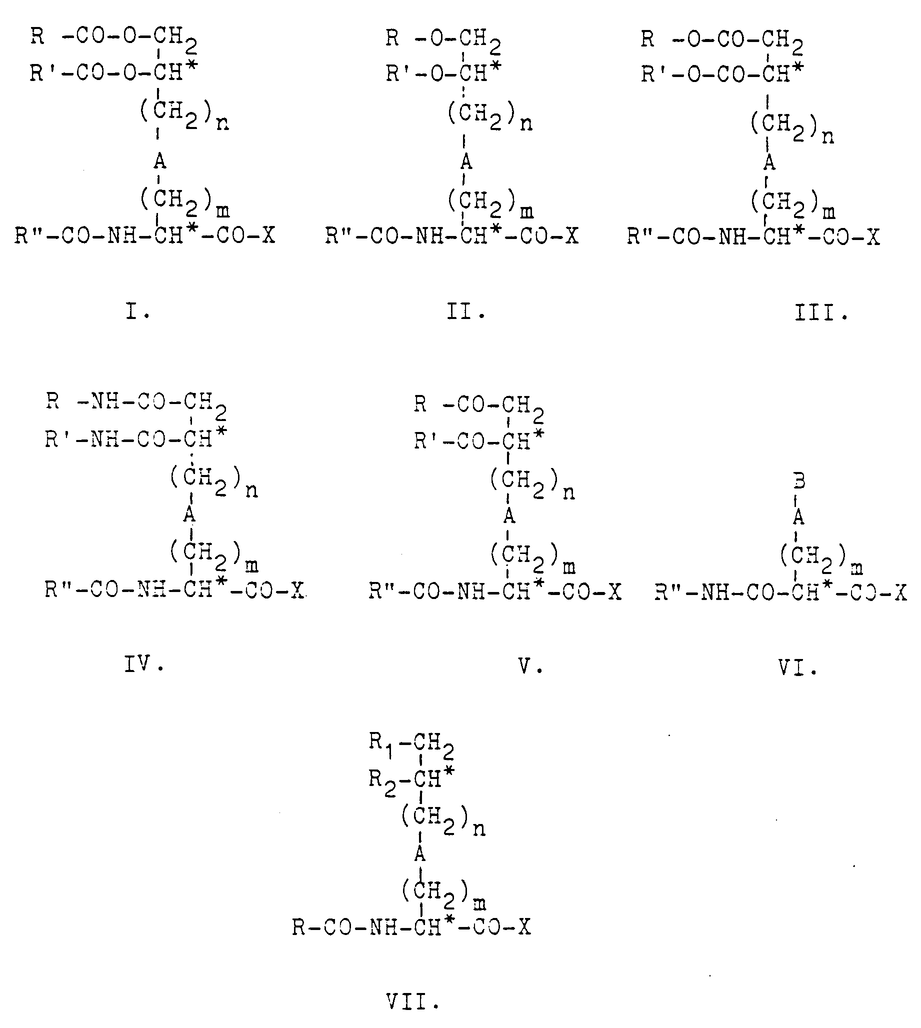 in denen A Schwefel, Sauerstoff, Disulfid (-S-S-),
Methylen (-CH₂-) oder -NH- sein kann;
n = 0 bis 5, m = 1 oder 2;
C* ein asymmetrisches Kohlenstoffatom mit R- oder S-Konfiguration,
R, R' und R'' gleich oder verschieden sind und Wasserstoff oder eine Alkyl-, Alkenyl- oder Alkinylgruppe mit 7 bis 25 Kohlenstoffatomen ist, welche mit Hydroxy-, Amino-, Oxo-, Acyl-, Alkyl- oder Cycloalkylgruppen substituiert sein kann, B in Formel VI die Bedeutung jedes der in den Formeln I - V aufgeführten -(CH₂)ₙ-(substituiertes Alkyl)-Reste haben kann und R₁ und R₂ gleich oder verschieden sind und dieselben Bedeutungen wie R, R' und R'' haben, aber auch -OR, -O-COR, -COOR, NHCOR oder -CONHR sein können, wobei X eine Kette von bis zu 10 Aminosäuren ist, an die die Partialsequenz des Virus gebunden ist.

2. Synthetische Vakzine nach
Anspruche 1 , dadurch gekennzeichnet, daß die Partialsequenz des Maul- und Klauenseuche-Virus, die an die Membranankerverbindung gebunden ist, ausgewählt ist aus der Gruppe
Sequenz-(134-154)
Sequenz-(135-154)
Sequenz-(134-158)
Sequenz-(134-160)
Sequenz-(141-160)
Sequenz-(141-158)
Sequenz-(200-213)
Sequenz-(200-210)
Sequenz-(161-180),
oder deren C-terminal amidierte oder alkylamidierte Formen, wobei die Sequenzen aller bekannten Serotypen und Subtypen verwendet werden können.

3. Synthetische Vakzine nach den Ansprüchen 1 oder 2, dadurch gekennzeichnet, daß die Partialsequenz des Maul- und Klauenseuche-Virus VP 1 (135-154) an die Membranankerverbindung gebunden ist.

4. Synthetische Vakzine nach einem oder mehreren der Ansprüche 1 - 3, dadurch gekennzeichnet, daß sie aus einer Mischung von Peptiden aus verschiendenen Sero- und/oder Subtypen des Maul- und Klauenseuche-Virus besteht, die jeweils kovalent an die Membranankerverbindung oder Membranankerverbindungen gebunden sind.

5. Synthetische Vakzine nach einem oder mehreren der Ansprüche 1 - 4, dadurch gekennzeichnet, daß sie aus einer Mischung von Sequenzen VP1 134 - 160 der Serotypen O, A oder C gebunden an die Membranankerverbindung N-Palmitoyl-S-[2,3-(bispalmitoyloxy)-propyl]-cysteinyl-seryl-serin besteht.

6. Synthetische Vakzine gemäß einem oder mehreren der Ansprüche 1 - 5, dadurch gekennzeichnet, daß die Vakzine aus N-Palmitoyl-S-[2,3-(bispalmitoyloxy)-propyl]cysteinyl-seryl-seryl VP 1 (135-154) besteht, wobei die Membranankerverbindung als R,S-, R,R-Diastereomeres oder als Diastereomerengemisch vorliegen kann.

7. Synthetische Vakzine gemäß einem oder mehreren der Ansprüche 1 bis 6, dadurch gekennzeichnet, daß die Membranankerverbindung als R,R-Diastereomeres vorliegt.

8. Verfahren zur Herstellung einer synthetischen Vakzine gemäß einem oder mehreren der Ansprüche 1 - 7 , dadurch gekennzeichnet, daß die in an sich bekannter Weise hergestellten Partialsequenzen des Maul- und Klauenseuche-Virus durch eine Konjugationsreaktion an die Membranankerverbindung gebunden werden.

9. Pharmazeutische oder veterinärmedizinische Zubereitung, gekennzeichnet durch einen Gehalt an einer synthetischen Vakzine gemäß einem oder mehreren der Ansprüche 1 - 8 gegebenenfalls neben üblichen Hilfs- und/oder Trägerstoffen, Adjuvanzien und/oder weiteren Vakzinen.

10. Verwendung einer synthetischen Vakzine gemäß einem oder mehreren der Ansprüche 1 - 9 zur Erzeugung von Antikörpern gegen Maul- und Klauenseuche-Viren in Säugetieren.

## Patentansprüche (Patentansprüche für folgende(n) Vertragsstaat(en): ES, GR)

1. Verfahren zur Herstellung einer synthetischen Vakzine, dadurch gekennzeichnet, daß die Vakzine eine Wirkung gegen die Maul- und Klauenseuche aufweist und mindestens eine Membranankerverbindung durch eine Konjugationsreaktion mit mindestens einer Partialsequenz eines Proteins des Maul- und Klaueseuche-Virus verbunden wird, wobei die Membranankerverbindung eine Struktur der nachfolgenden Formeln besitzt 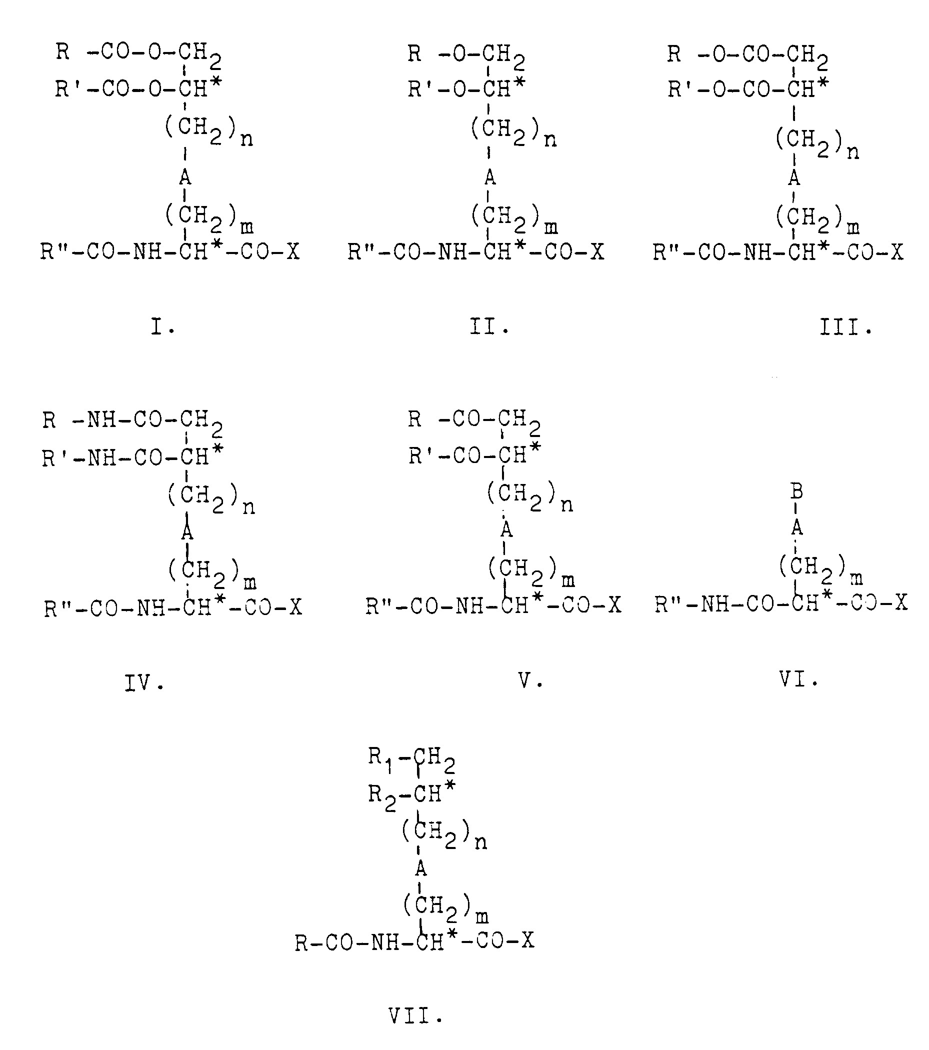 in denen A Schwefel, Sauerstoff, Disulfid (-S-S-),
Methylen (-CH₂-) oder -NH- sein kann;
n = 0 bis 5, m = 1 oder 2;
C* ein asymmetrisches Kohlenstoffatom mit R- oder S-Konfiguration,
R, R' und R'' gleich oder verschieden sind und Wasserstoff oder eine Alkyl-, Alkenyl- oder Alkinylgruppe mit 7 bis 25 Kohlenstoffatomen ist, welche mit Hydroxy-, Amino-, Oxo-, Acyl-, Alkyl- oder Cycloalkylgruppen substituiert sein kann, B in Formel VI die Bedeutung jedes der in den Formeln I - V aufgeführten -(CH₂)ₙ-(substituiertes Alkyl)-Reste haben kann und R₁ und R₂ gleich oder verschieden sind und dieselben Bedeutungen wie R, R' und R'' haben, aber auch -OR, -O-COR, -COOR, NHCOR oder -CONHR sein können, wobei X eine Kette von bis zu 10 Aminosäuren ist, an die die Partialsequenz des Virus gebunden ist.

2. Verfahren zur Herstellung einer synthetischen Vakzine nach Anspruch 1 , dadurch gekennzeichnet, daß die Partialsequenz des Maul- und Klauenseuche-Virus, die an die Membranankerverbindung gebunden ist, ausgewählt ist aus der Gruppe
Sequenz-(134-154)
Sequenz-(135-154)
Sequenz-(134-158)
Sequenz-(134-160)
Sequenz-(141-160)
Sequenz-(141-158)
Sequenz-(200-213)
Sequenz-(200-210)
Sequenz-(161-180),
oder deren C-terminal amidierte oder alkylamidierte Formen, wobei die Sequenzen aller bekannten Serotypen und Subtypen verwendet werden können.

3. Verfahren zur Herstellung einer synthetischen Vakzine nach den Ansprüchen 1 oder 2 dadurch gekennzeichnet, daß die Partialsequenz des Maul- und Klauenseuche-Virus VP 1 (135-154) an die Membranankerverbindung gebunden ist.

4. Verfahren zur Herstellung einer synthetischen Vakzine nach einem oder mehreren der Ansprüche 1 - 3, dadurch gekennzeichnet, daß eine Mischung von Peptiden aus verschiendenen Sero- und/oder Subtypen des Maul- und Klauenseuche-Virus jeweils kovalent an die Membranankerverbindung oder Membranankerverbindungen gebunden wird.

5. Verfahren zur Herstellung einer synthetischen Vakzine nach einem oder mehreren der Ansprüche 1 - 4, dadurch gekennzeichnet, daß eine Mischung von Sequenzen VP1 134 - 160 der Serotypen O, A oder C an die Membranankerverbindung N-Palmitoyl-s-[2,3-(bispalmitoyloxy)-propyl]-cysteinyl-seryl-serin gebunden wird.

6. Verfahren zur Herstellung einer synthetischen Vakzine gemäß einem oder mehreren der Ansprüche 1 - 5, dadurch gekennzeichnet, daß N-Palmitoyl-S-[2,3-(bispalmitoyloxy)-propyl]-cysteinyl-seryl-serin VP 1 (135-154) gebildet wird, wobei die Membranankerverbindung als R,S-, R,R-Diastereomeres oder als Diastereomerengemisch vorliegen kann.

7. Verfahren zur Herstellung einer synthetischen Vakzine gemäß einem oder mehreren der Ansprüche 1 bis 6, dadurch gekennzeichnet, daß die Membranankerverbindung als R,R-Diastereomeres vorliegt.

8. Verfahren zur Herstellung einer pharmazeutischen oder veterinärmedizinischen Zubereitung, dadurch gekennzeichnet, daß eine synthetische Vakzine hergestellt gemäß einem oder mehreren der Ansprüche 1 - 7 mit üblichen Hilfs- und/oder Trägerstoffen, Adjuvanzien und/oder weiteren Vakzinen in an sich bekannter Weise in eine zur Verabreichung geeignete Form gebracht wird.

9. Verwendung einer synthetischen Vakzine gemäß einem oder mehreren der Ansprüche 1 - 8 zur Erzeugung von Antikörpern gegen Maul- und Klauenseuche-Viren in Säugetieren.

## Claims (Claims for the following Contracting State(s): AT, BE, CH, DE, FR, GB, IT, LI, LU, NL, SE)

1. A synthetic vaccine which is active against foot and mouth disease and comprises a conjugate of a membrane-anchoring compound and a partial sequence of the foot and mouth disease virus which are linked together covalently where the membrane-anchoring compound has a structure from the formulae below 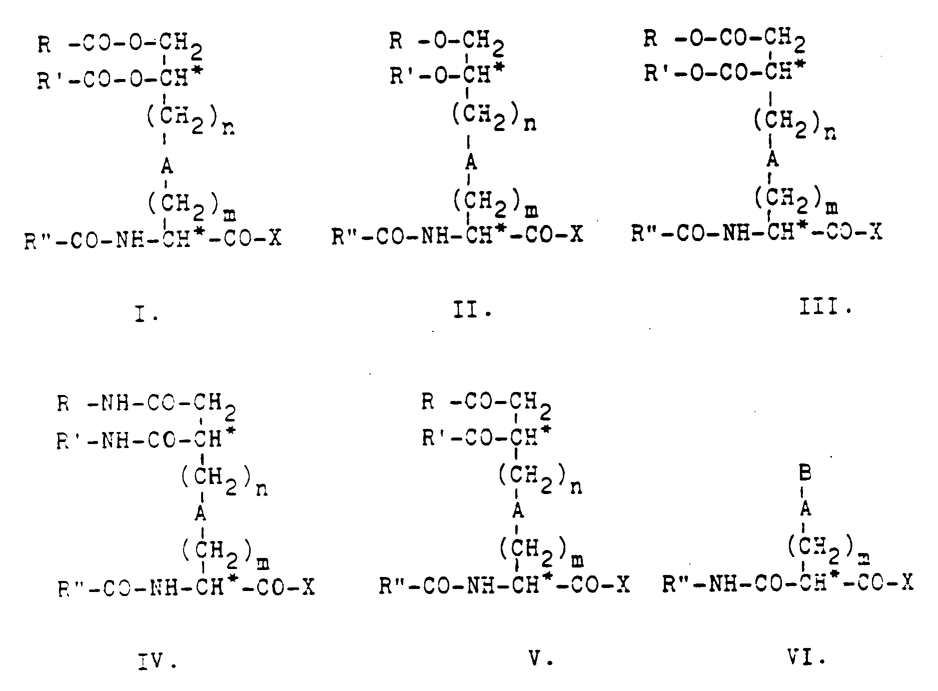 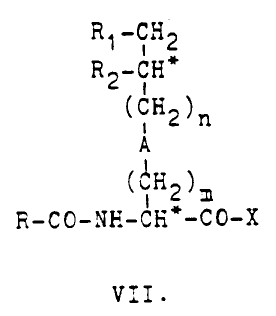 in which A can be sulfur, oxygen, disulfide
(-S-S-), methylene (-CH₂-) or -NH-;
n = 0 to 5, m = 1 or 2;
C* is an asymmetric carbon atom with the R or S configuration,
R, R' and R'' are identical or different and each is hydrogen or an alkyl, alkenyl or alkynyl group which has 7 to 25 carbon atoms and which can be substituted by hydroxyl, amino, oxo, acyl, alkyl or cycloalkyl groups, B in formula VI can have the meaning of each of the -(CH₂)ₙ-(substituted alkyl) radicals listed in formulae I-V, and R₁ and R₂ are identical or different and have the same meanings as R, R' and R'' but can also be -OR, -O-COR, -COOR, NHCOR or -CONHR, where X is a chain of up to 10 amino acids to which the partial sequence of the virus is bonded.

2. A synthetic vaccine as claimed in claim 1, wherein the partial sequence of the foot and mouth disease virus which is bonded to the membrane-anchoring compound is selected from the group comprising
sequence-(134-154)
sequence-(135-154)
sequence-(134-158)
sequence-(134-160)
sequence-(141-160)
sequence-(141-158)
sequence-(200-213)
sequence-(200-210)
sequence-(161-180),
or the C-terminal amidated or alkylamidated forms thereof, it being possible to use the sequences of all known serotypes and subtypes.

3. A synthetic vaccine as claimed in claim 1 or 2, wherein the partial sequence of the foot and mouth disease virus VP 1 (135-154) is bonded to the membrane-anchoring compound.

4. A synthetic vaccine as claimed in one or more of claims 1 - 3, which comprises a mixture of peptides from various sero- and/or subtypes of the foot and mouth disease virus, each of which is covalently bonded to the membrane-anchoring compound or membrane-anchoring compounds.

5. A synthetic vaccine as claimed in one or more of claims 1 - 4, which comprises a mixture of sequences VP1 134-160 of serotypes O, A or C bonded to the membrane-anchoring compound N-palmitoyl-S-[2,3-(bispalmitoyloxy)propyl]-cysteinyl-seryl-serine.

6. A synthetic vaccine as claimed in one or more of claims 1 - 5, which vaccine comprises N-palmitoyl-S-[2,3-(bispalmitoyloxy)propyl]cysteinyl-seryl-seryl-VP 1 (135-154), it being possible for the membrane-anchoring compound to be in the form of the R,S or R,R diastereomer or of a mixture of diastereomers.

7. A synthetic vaccine as claimed in one or more of claims 1 to 6, wherein the membrane-anchoring compound is in the form of the R,R diastereomer.

8. A process for the preparation of a synthetic vaccine as claimed in one or more of claims 1 - 7, which comprises the partial sequences of the foot and mouth disease virus which have been prepared in a manner known per se being bonded to the membrane-anchoring compound by a conjugation reaction.

9. A pharmaceutical or veterinary medicinal formulation, which contains a synthetic vaccine as claimed in one or more of claims 1 - 8, where appropriate in addition to customary auxiliaries and/or carriers, adjuvants and/or other vaccines.

10. The use of a synthetic vaccine as claimed in one or more of claims 1 - 9 for raising antibodies against foot and mouth disease viruses in mammals.

## Claims (Claims for the following Contracting State(s): ES, GR)

1. A process for the preparation of a synthetic vaccine, which is active against foot and mouth disease, and which comprises at least one membrane-anchoring compound being bonded by a conjugation reaction to at least one partial sequence of a protein of the foot and mouth disease virus, where the membrane-anchoring compound has a structure from the formulae below 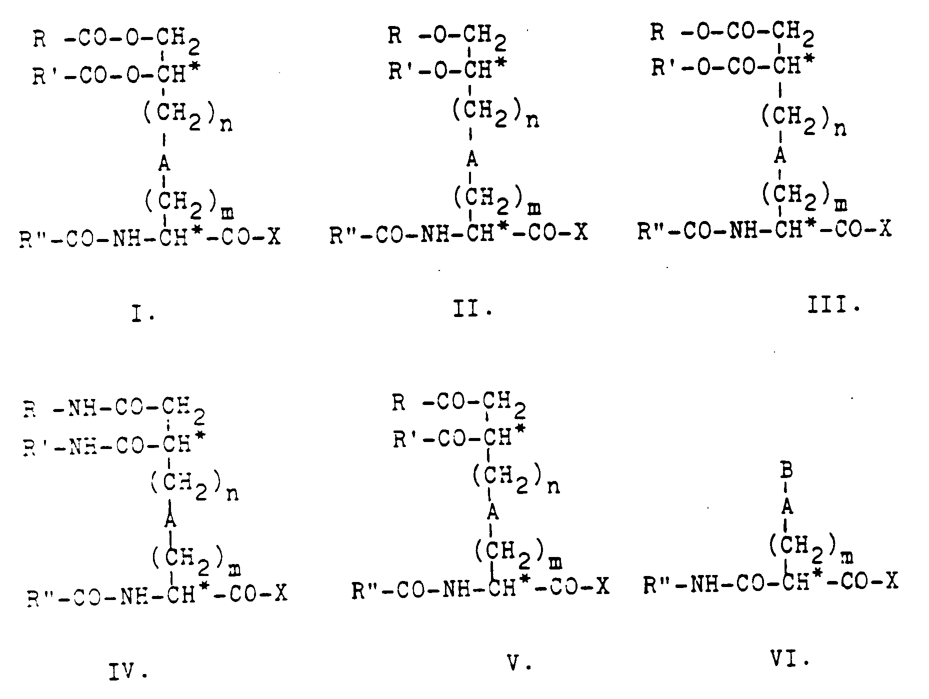 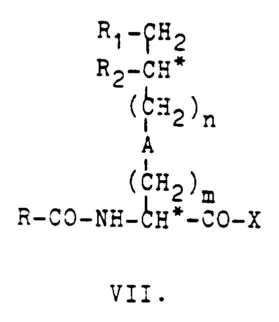 in which A can be sulfur, oxygen, disulfide
(-S-S-), methylene (-CH₂-) or -NH-;
n = 0 to 5, m = 1 or 2;
C* is an asymmetric carbon atom with the R or S configuration,
R, R' and R'' are identical or different and each is hydrogen or an alkyl, alkenyl or alkynyl group which has 7 to 25 carbon atoms and which can be substituted by hydroxyl, amino, oxo, acyl, alkyl or cycloalkyl groups, B in formula VI can have the meaning of each of the -(CH₂)ₙ-(substituted alkyl) radicals listed in formulae I-V, and R₁ and R₂ are identical or different and have the same meanings as R, R' and R'' but can also be -OR, -O-COR, -COOR, NHCOR or - CONHR, where X is a chain of up to 10 amino acids to which the partial sequence of the virus is bonded.

2. The process for the preparation of a synthetic vaccine as claimed in claim 1, wherein the partial sequence of the foot and mouth disease virus which is bonded to the membrane-anchoring compound is selected from the group comprising
sequence-(134-154)
sequence-(135-154)
sequence-(134-158)
sequence-(134-160)
sequence-(141-160)
sequence-(141-158)
sequence-(200-213)
sequence-(200-210)
sequence-(161-180),
or the C-terminal amidated or alkylamidated forms thereof, it being possible to use the sequences of all known serotypes and subtypes.

3. The process for the preparation of a synthetic vaccine as claimed in cliam 1 or 2, wherein the partial sequence of the foot and mouth disease virus VP 1 (135-154) is bonded to the membrane-anchoring compound.

4. The process for the preparation of a synthetic vaccine as claimed in one or more of claims 1 - 3, wherein a mixture of peptides from various sero- and/or subtypes of the foot and mouth disease virus is covalently bonded in each case to the membrane-anchoring compound or membrane-anchoring compounds.

5. The process for the preparation of a synthetic vaccine as claimed in one or more of claims 1 - 4, wherein a mixture of sequences VP1 134-160 of sero-types O, A or C is bonded to the membrane-anchoring compound N-palmitoyl-S-[2,3-(bispalmitoyloxy)propyl]cysteinyl-seryl-serine.

6. The process for the preparation of a synthetic vaccine as claimed in one or more of claims 1 - 5, wherein N-palmitoyl-S-[2,3-(bispalmitoyloxy)propyl]cysteinyl-seryl-seryl-VP1 (135-154) is formed, it being possible for the membrane-anchoring compound to be in the form of the R,S or R,R diastereomer or of a mixture of diastereomers.

7. The process for the preparation of a synthetic vaccine as claimed in one or more of claims 1 to 6, wherein the membrane-anchoring compound is in the form of the R,R diastereomer.

8. A process for the preparation of a pharmaceutical or veterinary medicinal formulation, which comprises a synthetic vaccine prepared as claimed in one or more of claims 1 - 7 being converted into a form suitable for administration with customary auxiliaries and/or carriers, adjuvants and/or other vaccines in a manner known per se.

9. The use of a synthetic vaccine as claimed in one or more of claims 1 - 8 for raising antibodies against foot and mouth disease viruses in mammals.

## Revendications (Revendications pour l'(les) Etat(s) contractant(s) suivant(s): AT, BE, CH, DE, FR, GB, IT, LI, LU, NL, SE)

1. Vaccin synthétique, caractérisé en ce que le vaccin a une action contre la fièvre aphteuse et consiste en un conjugué d'un composé de fixation à la membrane et d'une séquence partielle du virus de la fièvre aphteuse, qui sont liés l'un à l'autre par covalence, le composé de fixation à la membrane ayant une structure de formules suivantes 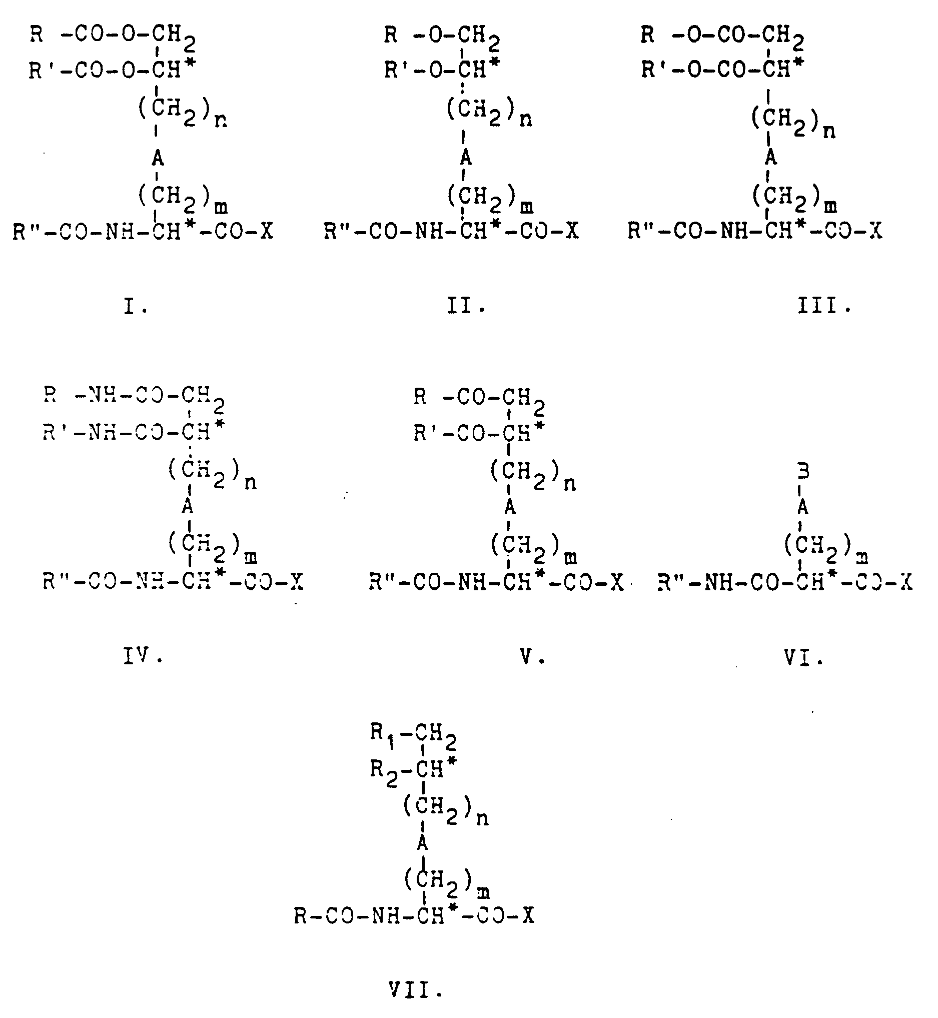 dans lesquelles A peut être un atome de soufre ou d'oxygène, un pont disulfure (-S-S-), un groupe méthylène (-CH₂-) ou -NH-; n va de 0 à 5, m = 1 ou 2;
C* est un atome de carbone asymétrique à configuration R ou S;
R, R' et R'' sont identiques ou différents et représentent un atome d'hydrogène ou un groupe alkyle, alcényle ou alcynyle ayant de 7 à 25 atomes de carbone, qui peut être substitué par des groupes hydroxy, amino, oxo, acyle, alkyle ou cycloalkyle;
B dans la formule VI peut avoir la signification de chacun des radicaux -(CH₂)ₙ-(alkyle substitué) indiqués dans les formules I-V;
et R₁ et R₂ sont identiques ou différents et ont les mêmes significations que R, R' et R'', mais peuvent également être -OR, -O-COR, -COOR, (-)NHCOR ou -CONHR, X étant une chaîne de 1 à 10 aminoacides, à laquelle est liée la séquence partielle du virus.

2. Vaccin synthétique selon la revendication 1, caractérisé en ce que la séquence partielle du virus de la fièvre aphteuse, qui est liée au composé de fixation à la membrane, est choisie parmi les suivantes
séquence -(134-154)
séquence -(135-154)
séquence -(134-158)
séquence -(134-160)
séquence -(141-160)
séquence -(141-158)
séquence -(200-213)
séquence -(200-210)
séquence -(161-180)
ou leurs formes amidées ou alkylamidées à l'extrémité C-terminale, les séquences de tous les sérotypes et sous-types connus pouvant être utilisées.

3. Vaccin synthétique selon la revendication 1 ou 2, caractérisé en ce que la séquence partielle VP1 (135-154) du virus de la fièvre aphteuse est liée au composé de fixation à la membrane.

4. Vaccin synthétique selon une ou plusieurs des revendications 1 à 3, caractérisé en ce qu'il consiste en un mélange de peptides provenant de divers sérotypes et/ou sous-types du virus de la fièvre aphteuse, qui sont liés chacun par covalence au composé de fixation à la membrane ou aux composés de fixation à la membrane.

5. Vaccin synthétique selon une ou plusieurs des revendications 1 à 4, caractérisé en ce qu'il consiste en un mélange de séquences VP1 (135-160 des sérotypes O, A ou C, liées au composé de fixation à la membrane N-palmitoyl-S-[2,3-(bis-palmitoyloxy)propyl]-cystéinyl-séryl-sérine.

6. Vaccin synthétique selon une ou plusieurs des revendications 1 à 5, caractérisé en ce que le vaccin consiste en N-palmitoyl-S-[2,3-(bis-palmitoyloxy)propyl]cystéinyl-séryl-séryl-VP1(135-154), le composé de fixation à la membrane pouvant se trouver sous forme de diastéréoisomère R,S, R,R ou sous forme d'un mélange de diastéréoisomères.

7. Vaccin synthétique selon une ou plusieurs des revendications 1 à 6, caractérisé en ce que le composé de fixation à la membrane se trouve sous forme de diastéréoisomère R,R.

8. Procédé pour la préparation d'un vaccin synthétique selon une ou plusieurs des revendications 1 à 7, caractérisé en ce que les séquences partielles du virus de la fièvre aphteuse, préparées d'une façon connue en soi, sont liées par une réaction de conjugaison au composé de fixation à la membrane.

9. Composition pharmaceutique ou pour médecine vétérinaire, caractérisée par une teneur en un vaccin synthétique selon une ou plusieurs des revendications 1 à 8, éventuellement en plus d'agents auxiliaires et/ou véhicules, adjuvants usuels et/ou d'autres vaccins.

10. Utilisation d'un vaccin synthétique selon une ou plusieurs des revendications 1 à 9, pour la production d'anticorps dirigés contre des virus de la fièvre aphteuse chez des mammifères.

## Revendications (Revendications pour l'(les) Etat(s) contractant(s) suivant(s): ES, GR)

1. Procédé pour la préparation d'un vaccin synthétique, caractérisé en ce que le vaccin a une action contre la fière aphteuse et en ce que au moins un composé de fixation à la membrane est lié par une réaction de conjugaison à au moins une séquence partielle d'une protéine du virus de la fièvre aphteuse, le composé de fixation à la membrane ayant une structure de formules suivantes 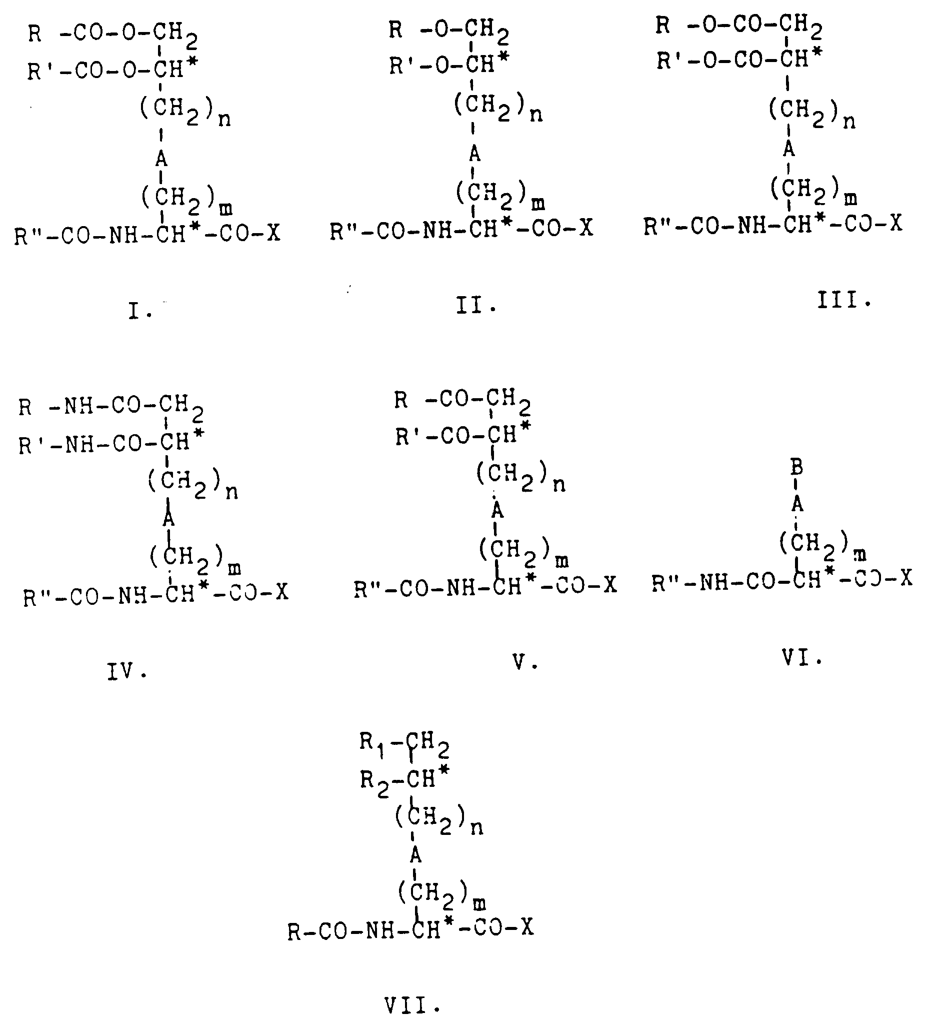 dans lesquelles A peut être un atome de soufre ou d'oxygène, un pont disulfure (-S-S-), un groupe méthylène (-CH₂-) ou -NH-; n va de 0 à 5, m = 1 ou 2;
C* est un atome de carbone asymétrique à configuration R ou S;
R, R' et R'' sont identiques ou différents et représentent un atome d'hydrogène ou un groupe alkyle, alcényle ou alcynyle ayant de 7 à 25 atomes de carbone, qui peut être substitué par des groupes hydroxy, amino, oxo, acyle, alkyle ou cycloalkyle;
B dans la formule VI peut avoir la signification de chacun des radicaux -(CH₂)ₙ-(alkyle substitué) indiqués dans les formules I-V;
et R₁ et R₂ sont identiques ou différents et ont les mêmes significations que R, R' et R'', mais peuvent également être -OR, -O-COR, -COOR, (-)NHCOR ou -CONHR, X étant une chaîne de 1 à 10 aminoacides, à laquelle est liée la séquence partielle du virus.

2. Procédé pour la préparation d'un vaccin synthétique selon la revendication 1, caractérisé en ce que la séquence partielle du virus de la fièvre aphteuse, qui est liée au composé de fixation à la membrane, est choisie parmi les suivantes
séquence -(134-154)
séquence -(135-154)
séquence -(134-158)
séquence -(134-160)
séquence -(141-160)
séquence -(141-158)
séquence -(200-213)
séquence -(200-210)
séquence -(161-180)
ou leurs formes amidées ou alkylamidées à l'extrémité C-terminale, les séquences de tous les sérotypes et sous-types connus pouvant être utilisées.

3. Procédé pour la préparation d'un vaccin synthétique selon la revendication 1 ou 2, caractérisé en ce que la séquence partielle VP1 (135-154) du virus de la fièvre aphteuse est liée au composé de fixation à la membrane.

4. Procédé pour la préparation d'un vaccin synthétique selon une ou plusieurs des revendications 1 à 3, caractérisé en ce qu'il consiste en un mélange de peptides provenant de divers sérotypes et/ou sous-types du virus de la fièvre aphteuse qui sont liés chacun par covalence au composé de fixation à la membrane ou aux composés de fixation à la membrane.

5. Procédé pour la préparation d'un vaccin synthétique selon une ou plusieurs des revendications 1 à 4, caractérisé en ce qu'un mélange de séquences VP1 134-160 des sérotypes O, A ou C, est lié au composé de fixation à la membrane N-palmitoyl-S-[2,3-(bis-palmitoyloxy)propyl]cystéinyl-séryl-sérine.

6. Procédé pour la préparation d'un vaccin synthétique selon une ou plusieurs des revendications 1 à 5, caractérisé en ce que l'on forme le conjugué N-palmitoyl-S-[2,3-(bis-palmitoyloxy)propyl]cystéinyl-séryl-séryl-VP1(135-154), le composé de fixation à la membrane pouvant se trouver sous forme de diastéréoisomères R,S, R,R ou sous forme d'un mélange de diastéréoisomères.

7. Procédé pour la préparation d'un vaccin synthétique selon une ou plusieurs des revendications 1 à 6, caractérisé en ce que le composé de fixation à la membrane se trouve sous forme de diastéréoisomère R,R.

8. Procédé pour la préparation d'une composition pharmaceutique ou pour médecine vétérinaire, caractérisé en ce que, d'une façon connue en soi, on met sous une forme appropriée à l'administration un vaccin synthétique préparé selon une ou plusieurs des revendications 1 à 7, avec des agents auxiliaires et/ou véhicules, adjuvants usuels et/ou d'autres vaccins.

9. Utilisation d'un vaccin synthétique selon une ou plusieurs des revendications 1 à 8, pour la production d'anticorps dirigés contre des virus de la fièvre aphteuse chez des mammifères.
